# EUROPÄISCHE PATENTANMELDUNG

(11) **EP 0 642 018 A1**
(43) Veröffentlichungstag der Anmeldung: **08.03.1995**
(21) Anmeldenummer: 94113849.7
(22) Anmeldetag: 03.09.1994
(51) Int. Cl.: G01N 33/18

(54) **Vorrichtung zum Nachweis von in Wasser dispergiertem Öl**

(30) Priorität: 07.09.1993 DE 4330301
(71) Anmelder: GRUNDIG E.M.V. Elektro-Mechanische Versuchsanstalt Max Grundig GmbH & Co. KG, D-90762 Fürth (DE)
(72) Erfinder: Hallas, Ernst, Dr., c/o Grundig E.M.V., D-90748 Fürth (DE)

(57) **Zusammenfassung**

Die Anmeldung betrifft ein Verfahren und eine Vorrichtung zur Durchführung des Verfahrens zum Nachweis von in Wasser dispergiertem Öl.

Aufgabe ist es, ein Verfahren und eine Vorrichtung zum Nachweis von in Wasser dispergiertem Öl anzugeben, bei dem bereits geringste Mengen von Öl im Wasser erkannt werden können, wobei durch die Messung eine allgemeingültige Aussage über den Zustand des Wassers erhalten werden soll.

Die Abgasung des einlaufenden Mediums, also des Ab- oder Prozeßwassers, wird bereichsweise von der Umwelt abgeschlossen. Die abgeschlossene Abgasung wird dann mittels einer Meßsonde auf den Gehalt an nichtflüchtigen Kohlenwasserstoffen hin untersucht.

## Beschreibung

Die Erfindung betrifft eine Vorrichtung zum Nachweis von in Wasser dispergiertem und/oder auf der Wasseroberfläche schwimmenden Öl nach dem Oberbegriff des Anspruches 1.

Insbesondere Flüsse in der Nähe von Industrie- oder Kraftwerksanlagen, in die Abwässer dieser Anlagen eingeleitet werden, oder Zu- bzw. Abläufe von Klärwerken müssen regelmäßig oder ständig auf Fremdstoffe untersucht werden. Besonders die Öldetektion spielt hierbei eine große Rolle, da eine Verschmutzung mit Öl weitreichende negative Folgen hat.

Zur Erkennung von Verschmutzungen des Wassers durch Öl oder sonstige Fremdstoffe werden häufig Trübungsmeßgeräte eingesetzt. Dazu werden dem Gewässer Proben entnommen, von denen die Trübungswerte gemessen werden, die auf den Grad der Verschmutzung schließen lassen. Die Art der Verschmutzung kann jedoch mit dieser Methode nicht ermittelt werden, da beispielsweise durch Öl verursachte Verschmutzung die gleichen Trübungswerte liefern kann wie eine durch aufgewühlten Schlamm verursachte Verschmutzung.

Ein Anzeigegerät zum Nachweis der Verschmutzung durch Öl oder ähnliche Verunreinigungen ist in der deutschen Offenlegungsschrift DE 22 24 310 A1 beschrieben. Dort werden ein oder mehrere Grenzflächen mit einer zu überwachenden Flüssigkeit auf der einen Seite benetzt, während auf der anderen Seite, in einem anderen Medium, ein Strahlenbündel einfällt, dessen Einfallswinkel so gewählt wird, daß er nahe am Grenzwinkel zur Totalreflexion liegt. Bei Vorhandensein von Öl in der zu untersuchenden Flüssigkeit wird der Grenzwinkel überschritten und es findet Totalreflexion statt, so daß eine Signalvorrichtung vom reflektierenden Strahlenbündel betätigt wird.
Bei einem derartigen Anzeigegerät besteht aber der Nachteil, daß jeweils nur eine Probe entnommen wird, die nicht zwingend eine allgemeingültige Aussage über den momentanen Zustand des Gewässers zuläßt.

In der deutschen Offenlegungsschrift DE 30 38 107 A1 ist ein Verfahren zur berührungsfreien Ermittlung von Öl auf einer Wasseroberfläche beschrieben. Die zu untersuchende Stelle der Wasseroberfläche wird mit einer ultravioletten Lichtquelle bestrahlt, die die Fluoreszenz der erwarteten Ölgruppe anregen soll. Das durch die Fluoreszenzwirkung abgestrahlte Licht wird fotoelektrisch empfangen, wobei die von der Oberfläche reflektierte, durch das UV-Licht der ultravioletten Lichtquelle im wesentlichen sperrende Filter geführte Strahlung, sowie die von der Fluoreszenz herrührende Oberflächenstrahlung vom fotoelektrischen Empfänger aufgenommen werden und die Gesamtintensität der ungerichteten Fluoreszenzstrahlung erfaßt wird.
Der Nachteil dieses Verfahrens liegt in der punktuellen Messung, bei der nicht eine großflächige Oberfläche überprüft wird, sondern nur ein relativ kleiner Bereich. Ferner kann mit diesem Verfahren nur ein Oberflächenbelag nachgewiesen werden, während geringere Verunreinigungen mit Öl, insbesondere in Wasser gelöstem Öl, mit diesem und auch den anderen Verfahren nicht ohne weiteres nachgewiesen werden können.

Aus der US-Patentschrift US-PS 3 957 439 ist ein Vefahren bekannt, bei dem Proben von Grundwasser auf Verunreinigungen durch Kohlenwasserstoffe untersucht werden. Die zu untersuchenden Proben werden durch eine Pumpe kontinuierlich entnommen und einer Meßvorrichtung zugeführt.
Der Nachteil dieser Anordnung besteht darin, daß auf dem Weg von der Probenentnahmestelle zur Meßeinrichtung chemische Veränderungen eintreten können, die das Meßergebnis verfälschen.

Aufgabe der Erfindung ist es deshalb, eine Vorrichtung zum Nachweis von insbesondere in Wasser dispergiertem Öl anzugeben, bei dem bereits geringste Mengen von Öl im Wasser erkannt werden können, wobei durch die Messung eine allgemeingültige Aussage über den Zustand des Wassers erhalten werden soll.

Diese Aufgabe wird ausgehend von den Merkmalen des Oberbegriffes des Anspruches 1 durch die kennzeichnenden Merkmale des Anspruches 1 gelöst.
Vorteilhafte Ausgestaltungen der Erfindung sind in den abhängigen Ansprüchen angegeben.

Gemäß der Erfindung wird die Abgasung des einlaufenden Mediums, also des Ab- oder Prozeßwassers, bereichsweise von der Umwelt abgeschlossen. Die abgeschlossene Abgasung wird dann mittels einer Meßsonde auf den Gehalt an nichtflüchtigen Kohlenwasserstoffen hin untersucht.

Durch die Abgrenzung der Abgasung an der Wasseroberfläche von der Umwelt werden die über der Wasseroberfläche entstehenden Gase nicht durch die Umgebungsatmosphäre verdünnt, so daß bereits geringste Mengen an Kohlenwasserstoffen gemessen werden können.

Eine Verbesserung der Ansprechempfindlichkeit wird erreicht, wenn unterhalb der Meßsonde in Bodennähe des Ab- bzw. Prozeßwasserkanales ein Belüftungselement angeordnet wird, dessen Belüftungswirkung an die vorgegebenen Strömungsverhältnisse angepasst wird. Das Belüftungselement wird dabei so angeordnet, daß die Abgasung aus dem Belüftungselement bei der Meßsonde austritt.

Durch die Belüftung wird der wesentliche Anteil der dispergierten, leicht flüchtigen Kohlenwasserstoffe ausgestrippt. Dadurch wird im Meßgas verstärkt auch die zur Flüssigkeit, entsprechend dem Partialdruck, im Gleichgewicht stehende Gasphase der Kohlenwasserstoffe aufgefangen.

Die erfindungsgemäße Vorrichtung beinhaltet gemäß einer weiteren vorteilhaften Ausgestaltung einen Kompressor, mit dem Druckluft über einen Zuführungskanal in den Belüftungskörper eingebracht wird.

Eine weitere vorteilhafte Ausgestaltung der erfindungsgemäßen Vorrichtung sieht vor, daß der Belüftungskörper unter einem vorgebbaren Winkel gegenüber der Richtung der Strömungsgeschwindigkeit angeordnet ist. Der Winkel wird in Abhängigkeit der Füllstandshöhe und der Strömungsgeschwindigkeit, die im Ab- oder Prozeßwasserkanal vorherrscht, so gewählt, daß die Abgasung in die Meßsonde maximal wird.

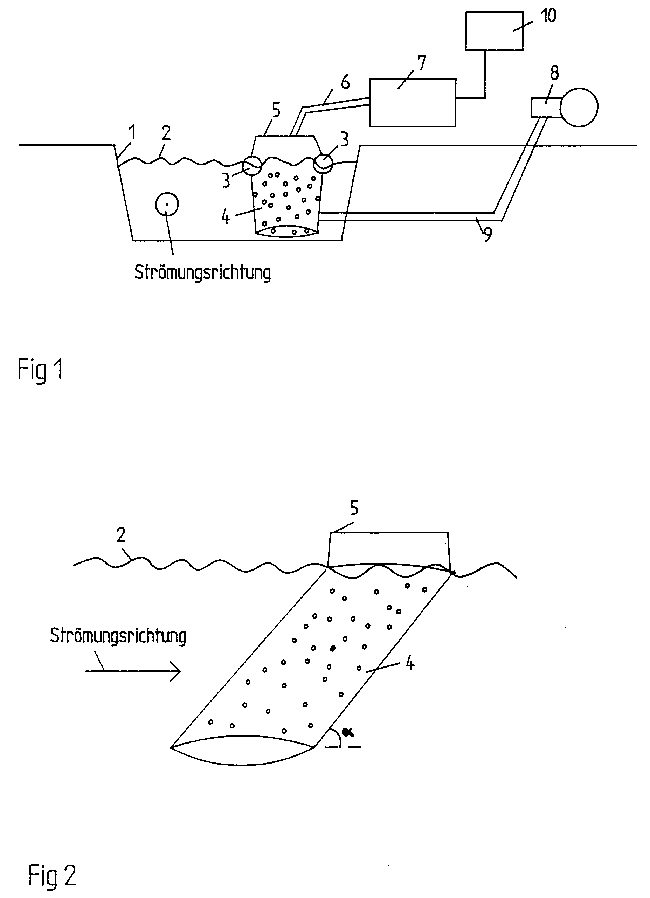

Im folgenden wird die Erfindung an Hand der Figuren 1 und 2 näher erläutert.

Es zeigen:
Figur 1 eine erfindungsgemäße Vorrichtung zum Nachweis von Öl in Wasser, und
Figur 2 eine Seitenansicht des Belüftungselementes.

In Figur 1 ist ein Ab- bzw. Prozeßwasserkanal 1 dargestellt. In diesem Ab- bzw. Prozeßwasserkanal ist das Belüftungselement 4 eingebracht, das beispielsweise aus einer Membranplatte besteht. Dieses Belüftungselement wird mittels schwimmfähiger Elemente 3, die auf der Wasseroberfläche liegen, festgehalten. Die schwimmfähigen Elemente 3 werden mittels Befestigungsvorrichtungen bekannter Art, die in der Zeichnung nicht dargestellt sind, in ihrer Position gehalten.

Über der Wasseroberfläche ist, ebenfalls an den schwimmfähigen Elementen 3, die Meßsonde 5 angebracht. Diese ist so ausgeführt, daß sie den Abgasungsbereich von der Umwelt abschirmt, um die Vermischung der Abgasung mit der Umgebungsatmosphäre zu verhindern.
Von der Meßsonde 5 führt eine Verbindungsleitung zu einem Meßgerät 7, mit dem der Kohlenwasserstoffanteil der Abgasung auf bekannte Art bestimmt wird.

Zur Verbesserung der Abgasungsbedingungen wird mittels eines Kompressors 8 Druckluft über einen Druckluftkanal 9 in das Belüftungselement 4 geblasen. Durch diese Belüftung im Belüftungselement 4 wird die Ausgasung von leicht flüchtigen Kohlenwasserstoffen verstärkt, wodurch sich eine höhere Meßgenauigkeit bzw. ein schnelleres Ansprechen auf dispergiertes Öl im Ab- bzw. Prozeßwasser ergibt.
Das Meßgerät 7 ist mit einer Anzeige- oder Alarmeinheit 10 verbunden. Eine Alarmausgabe kann in vorteilhafter Weise stufenweise erfolgen, indem für die Kohlenwasserstoffkonzentration verschiedene Schwellwerte vorgegeben werden, bei deren Überschreiten ein charakteristisches Alarmsignal ausgegeben wird. Es kann beispielsweise ein erster Schwellwert vorgegeben werden, bei dem ein Handeln nicht unbedingt erforderlich ist. Ein zweiter Schwellwert kann z.B. eine für biologische Prozesse kritische Konzentration sein und ein dritter Schwellwert kann eine Konzentration anzeigen, deren Überschreitung einen explosiven Charakter der Abgasung nicht ausschließt.

Figur 2 zeigt eine zu Figur 1 um 90^{o} gedrehte Ansicht, wobei dort nur das Belüftungselement 4 und die Meßsonde 5 dargestellt ist. Das Belüftungselement ist gemäß dieser Figur unter dem Winkel α gegenüber der Strömungsrichtung angeordnet. Der Winkel α wird in Abhängigkeit der vorherrschenden Verhältnisse (Füllstandshöhe, Strömungsgeschwindigkeit, usw.) so gewählt, daß die Verstärkung der Abgasung einen optimalen Wert erreicht. Der Winkel α wird in vorteilhafter Weise durch Vergleichsversuche ermittelt.

## Patentansprüche

1. Vorrichtung zum Nachweis von in Wasser dispergiertem und/oder auf der Wasseroberfläche schwimmenden Öl,
**dadurch gekennzeichnet**, daß
- in den Ab- oder Prozeßwasserkanal (1) unter der Wasseroberfläche (2) ein Belüftungskörper (4) eingebracht ist, und
- über der Wasseroberfläche eine Meßsonde (5) im Bereich des Belüftungskörpers (4) angeordnet ist, die über ein Verbindungselement (6) mit einem Meßgerät (7) verbunden ist.

2. Vorrichtung nach Anspruch 1,
**dadurch gekennzeichnet**, daß
der Belüftungskörper (4) und die Meßsonde (5) an schwimmfähigen Elementen (3) gelagert sind.

3. Vorrichtung nach Anspruch 1 oder 2,
**dadurch gekennzeichnet**, daß
unterhalb der Meßsonde in Bodennähe des Ab- oder Prozeßwasserkanales ein Belüftungselement angeordnet ist, wobei die Belüftung an die vorgegebenen Strömungsverhältnisse angepaßt wird und die Ausgasung aus dem Belüftungselement bei der Meßsonde austritt.

4. Vorrichtung nach Anspruch 3,
**dadurch gekennzeichnet**, daß
das Belüftungselement aus einem Kompressor (8) und einem Kanal (9) besteht, wobei über den Kanal (9) Druckluft in den Belüftungskörper (4) eingebracht wird.

5. Vorrichtung einem der Ansprüche 1 bis 4,
**dadurch gekennzeichnet**, daß
der Belüftungskörper (4) unter einem vorgebbaren Winkel α gegenüber der Strömungsrichtung angeordnet ist, wobei der Winkel in Abhängigkeit der Füllstandshöhe und der Strömungsgeschwindigkeit im Ab- oder Prozeßwasserkanal gewählt wird.
